# EUROPEAN PATENT APPLICATION

(11) **EP 4 834 414 A2**
(43) Date of publication of application: **04.11.2026**
(21) Application number: 26205832.4
(22) Date of filing: 24.02.2023
(51) Int. Cl.: A61M 39/20

(54) **DUAL CAVITY HEMOSTASIS VALVE HUB FOR INTRODUCER SHEATHS**

(30) Priority: 25.02.2022 US 202263314096 P
(62) Document number(s) of the earlier application(s) in accordance with Art. 76 EPC: 23713468.9 / 4 482 562
(71) Applicant: Boston Scientific Scimed Inc., Maple Grove, Minnesota 55311 (US); Boston Scientific Medical Device Ltd., Galway (IE)
(72) Inventor: SHARMA, Neeraj Kumar, 110058 New Delhi (IN); AGRAWAL, Sumit, 122001 Gurgaon Haryana (IN); PAHWA, Rupesh Kumar, 122018 Gurugram (IN); LIU, Qian, Plymouth, Minnesota, 55447 (US)
(74) Representative: Peterreins Schley

(57) **Abstract**

A hemostasis valve hub for an introducer sheath includes a hub base, a hub cap configured for engagement with the hub base, the hub cap having a first wall with a first opening and a second opening extending through the first wall, a manifold positioned within the hub cap and adjacent the hub base, a primary seal positioned adjacent the first opening of the hub cap, a secondary seal positioned adjacent the second opening of the hub cap, and wherein the primary seal and the secondary seal are configured to provide a fluid seal between the first opening and the second opening, respectively, and the hub base.

## Description

### CROSS-REFERENCE TO RELATED APPLICATIONS

This application claims priority to Provisional Application No. 63/314,096, filed February 25, 2022, which is herein incorporated by reference in its entirety.

### TECHNICAL FIELD

The present disclosure relates to a hub for an introducer sheath. More specifically, the present disclosure relates to a dual cavity hemostasis valve hub for large bore introducer sheaths.

### BACKGROUND

In various procedures for delivering intravascular medical devices, an introducer sheath is inserted into a blood vessel of a patient, for example a femoral artery, and medical devices are inserted into the introducer sheath for introduction into the blood vessel. In various instances, the medical devices include catheters or other medical devices such as a blood pump. In various instances, multiple medical devices need to be introduced into or inserted through the blood vessel at the same time. A hemostasis valve hub may be incorporated at a proximal end of the large bore introducer sheath to reduce blood leakage as devices are being inserted, positioned, and removed. There is a need for an optimized hemostasis valve hub that allows for the passage of multiple devices into the introducer sheath while still minimizing blood leakage and facilitating passage of the device through an introducer sheath.

### SUMMARY

In an Example 1, a hemostasis valve hub for an introducer sheath includes a hub base, a hub cap configured for engagement with the hub base, the hub cap having a first wall with a first opening and a second opening extending through the first wall, a manifold positioned within the hub cap and adjacent the hub base, a primary seal positioned adjacent the first opening of the hub cap, a secondary seal positioned adjacent the second opening of the hub cap, and wherein the primary seal and the secondary seal are configured to provide a fluid seal between the first opening and the second opening, respectively, and the hub base.

In an Example 2, the hemostasis valve hub of Example 1 includes wherein the first opening has a first diameter and the second opening has a second diameter, the first diameter being greater than the second diameter.

In an Example 3, the hemostasis valve hub of Examples 1 or 2 further includes wherein the primary seal has a first thickness, the secondary seal has a second thickness, and the first thickness is greater than the second thickness.

In an Example 4, the hemostasis valve hub of any one of Examples 1-3 further includes wherein the hub base includes a distal end and a proximal end and wherein the hub base has a ferrule positioned within the distal end of the hub base.

In an Example 5, the hemostasis valve hub of Example 4 further includes wherein the ferrule is configured for securing the introducer sheath with the hub base.

In an Example 6, the hemostasis valve hub of any one of Examples 1-3 further includes wherein the hub base comprises a distal end and a proximal end and wherein a threaded cap is configured for attachment to the distal end of the hub base for securing the introducer sheath with hub base.

In an Example 7, the hemostasis valve hub of any one of Examples 1-6 further includes wherein the primary seal and the secondary seal are each composed of cylindrical seals having a partial cross slit within a center of each the first seal and the second seal.

In an Example 8, the hemostasis valve hub of any one of Examples 1-7 further includes wherein the hub base includes at least one ring configured for receiving a suture to secure the hemostasis valve hub positioning.

In an Example 9, a delivery system for a plurality of medical devices into a blood vessel includes an introducer sheath for insertion into the blood vessel, the introducer sheath having a proximal end and a distal end, a hemostasis valve hub for attachment to the proximal end of the introducer sheath, the hemostasis valve hub including a hub base, a hub cap configured for engagement with the hub base, the hub cap having a first face with a first opening and a second opening extending through the first face, a manifold positioned within the hub cap and adjacent the hub base, a primary seal positioned adjacent the first opening of the hub cap, and a secondary seal positioned adjacent the second opening of the hub cap. The primary seal and the secondary seal are configured to provide a fluid seal between the first opening and the second opening, respectively, and the hub base.

In an Example 10, the delivery system of Example 9 further includes wherein the first opening has a first diameter and the second opening has a second diameter, the first diameter being greater than the second diameter.

In an Example 11, the delivery system of Example 9 or Example 10 further includes wherein the hub base has at least one ring for receiving a suture.

In an Example 12, the delivery system of any one of Examples 9-11 further includes wherein the hub base includes a distal end and a proximal end and wherein the hub base has a ferrule positioned within the distal end of the hub base.

In an Example 13, a method of assembling an introducer sheath and a hub includes placing a hub base onto the proximal end of the introducer sheath, attaching a securing mechanism to the hub base and the proximal end of the introducer sheath, and engaging a hub cap onto the hub base, the hub cap having a seal manifold positioned within the hub cap and a first face having a first opening and a second opening, a first seal positioned between the seal manifold and the first opening, and a second seal positioned between the seal manifold and the second opening.

In an Example 14, the method of Example 13 further includes wherein engaging the hub cap with the hub base includes aligning a first opening of the seal manifold with the primary seal and aligning a second opening of the seal with the secondary seal.

In an Example 15, the method of Example 14 further includes wherein the first opening of the seal manifold and the first opening of the hub cap define a first lumen, and the second opening of the seal manifold and the second opening of the hub cap define a second lumen.

In an Example 16, a hemostasis valve hub for an introducer sheath includes a hub base, a hub cap configured for engagement with the hub base, the hub cap having a first wall with a first opening and a second opening extending through the first wall, a manifold positioned within the hub cap and adjacent the hub base, a primary seal positioned adjacent the first opening of the hub cap, a secondary seal positioned adjacent the second opening of the hub cap, and wherein the primary seal and the secondary seal are configured to provide a fluid seal between the first opening and the second opening, respectively, and the hub base.

In an Example 17, the hemostasis valve hub of Example 16 includes wherein the first opening has a first diameter and the second opening has a second diameter, the first diameter being greater than the second diameter.

In an Example 18, the hemostasis valve hub of Example 16 further includes wherein the primary seal has a first thickness, the secondary seal has a second thickness, and the first thickness is greater than the second thickness.

In an Example 19, the hemostasis valve hub of Example 16 further includes wherein the hub base comprises a distal end and a proximal end and wherein the hub base has a ferrule positioned within the distal end of the hub base.

In an Example 20, the hemostasis valve hub of Example 19 further includes wherein the ferrule is configured for securing the introducer sheath with hub base.

In an Example 21, the hemostasis valve hub of Example 16 further includes wherein the hub base comprises a distal end and a proximal end and wherein a threaded cap is configured for attachment to the distal end of the hub base for securing the introducer sheath with hub base.

In an Example 22, the hemostasis valve hub of Example 16 further includes wherein the primary seal and the secondary seal are each composed of cylindrical seals having a partial cross slit within a center of each the first seal and the second seal.

In an Example 23, the hemostasis valve hub of Example 16 further includes wherein the hub base comprises at least one ring configured for receiving a suture to secure the hemostasis valve hub positioning.

In an Example 24, the hemostasis valve hub of Example 16 further includes wherein the hub base comprises an access port extending radially outward from the hub base.

In an Example 25, a delivery system for a plurality of medical devices into a blood vessel includes an introducer sheath for insertion into the blood vessel, the introducer sheath having a proximal end and a distal end, a hemostasis valve hub for attachment to the proximal end of the introducer sheath, the hemostasis valve hub including a hub base, a hub cap configured for engagement with the hub base, the hub cap having a first face with a first opening and a second opening extending through the first face, a manifold positioned within the hub cap and adjacent the hub base, a primary seal positioned adjacent the first opening of the hub cap, and a secondary seal positioned adjacent the second opening of the hub cap. The primary seal and the secondary seal are configured to provide a fluid seal between the first opening and the second opening, respectively, and the hub base.

In an Example 26, the delivery system of Example 25 further includes wherein the first opening has a first diameter and the second opening has a second diameter, the first diameter being greater than the second diameter.

In an Example 27, the delivery system of Example 25 further includes wherein the hub base has at least one ring for receiving a suture.

In an Example 28, the delivery system of Example 25 further includes wherein the hub base comprises a distal end and a proximal end and wherein the hub base has a ferrule positioned within the distal end of the hub base.

In an Example 29, the delivery system of Example 25 further includes wherein the hub base comprises a distal end and a proximal end and wherein a threaded cap is configured for attachment to the distal end of the hub base for securing the introducer sheath with hub base.

In an Example 30, a method of assembling an introducer sheath and a hub includes placing a hub base onto the proximal end of the introducer sheath, attaching a securing mechanism to the hub base and the proximal end of the introducer sheath, and engaging a hub cap onto the hub base, the hub cap having a seal manifold positioned within the hub cap and a first face having a first opening and a second opening, and a first seal positioned between the seal manifold and the first opening and a second seal positioned between the seal manifold and the second opening.

In an Example 31, the method of Example 30 further includes wherein the securing mechanism is a ferrule and the ferrule is positioned within the hub base.

In an Example 32, the method of Example 30 further includes wherein the securing mechanism is a threaded cap engaged with a distal end of the hub base.

In an Example 33, the method of Example 30 further includes wherein engaging the hub cap with the hub base includes aligning a first opening of the seal manifold with the primary seal and aligning a second opening of the seal with the secondary seal.

In an Example 34, the method of Example 33 further includes wherein the first opening of the seal manifold and the first opening of the hub cap define a first lumen, and the second opening of the seal manifold and the second opening of the hub cap define a second lumen.

In an Example 35, the method of Example 30 further includes wherein the first seal is a cylindrical seal having a partial cross slit within a center of the first seal.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 illustrates a cross sectional view of an introducer sheath after insertion into a blood vessel, in accordance with embodiments of the present disclosure.
FIG. 2 illustrates a cross sectional view of the introducer sheath after insertion into a blood vessel and insertion of a medical device into the introducer sheath, in accordance with embodiments of the present disclosure.
FIG. 3 illustrates a side view of a hemostasis valve hub for use with an introducer sheath, in accordance with embodiments of the present disclosure.
FIG. 4 illustrates a cross-sectional view of the hemostasis valve hub of FIG. 3, in accordance with embodiments of the present disclosure.
FIG. 5 is an enlarged view of a portion of a hemostasis valve hub, in accordance with embodiments of the present disclosure.
FIG. 6 is an exploded view of a hemostasis valve hub, in accordance with embodiments of the present disclosure.
FIG. 7A is a top view of a seal for use with a hemostasis valve hub, in accordance with embodiments of the present disclosure.
FIG. 7B is a cross sectional view of the seal of Fig. 7A, in accordance with embodiments of the present disclosure.
FIG. 7C is an additional cross sectional view of the seal of FIG. 7A, in accordance with present embodiments.
FIG. 7D is a top view of a seal for use with a hemostasis valve hub, in accordance with embodiments of the present disclosure.
FIG. 7E is a cross sectional view of the seal of Fig. 7D, in accordance with embodiments of the present disclosure.
FIG. 7F is an additional cross sectional view of the seal of FIG. 7D, in accordance with present embodiments.
FIG. 8 is an exploded view of a hemostasis valve hub, in accordance with embodiments of the present disclosure.
FIG. 9 is a flow chart illustrating a method of assembling an introducer sheath with a hub, in accordance with embodiments of the present disclosure.

### DETAILED DESCRIPTION

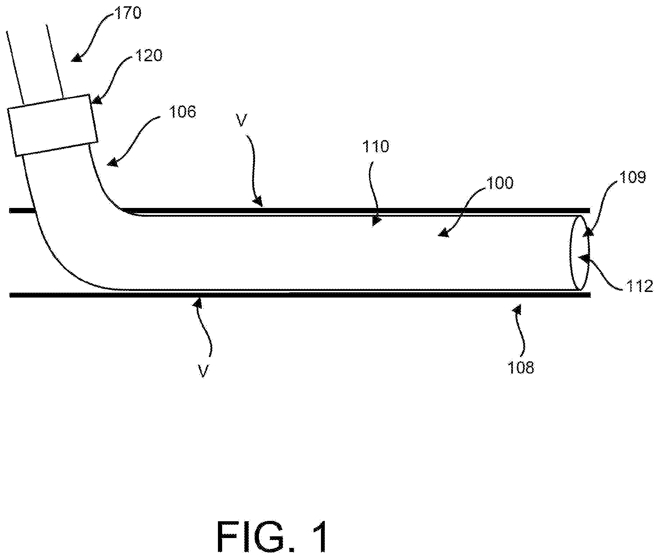

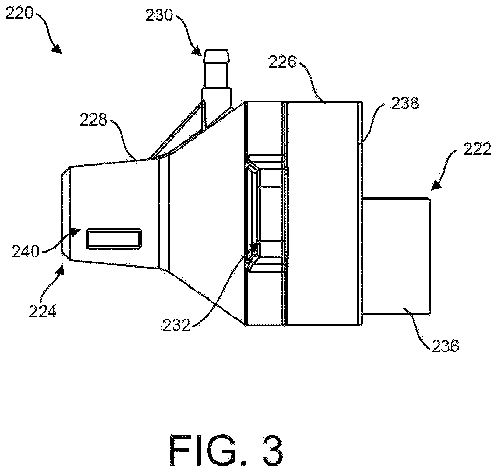

FIG. 1 illustrates a side cross sectional view of a blood vessel V with an introducer sheath 100, inserted at least partially into the blood vessel V. In some embodiments, the introducer sheath 100 is used for facilitating the passage of various relatively large medical devices, such as a blood pump as will be described further herein, through the introducer sheath 100 and into the blood vessel V. Hence, the introducer sheath 100 may be referred to as a large bore introducer sheath. The introducer sheath 100 comprises a proximal end 106 and a distal end 108 that is opposite the proximal end 106. The introducer sheath 100 includes a proximal opening (not shown) adjacent the proximal end 106 and a distal opening 109 adjacent the distal end 108. A body portion 110 of the introducer sheath 100 extends between the proximal end 106 and the distal end 108, and the body portion 110 defines a lumen 112 of the introducer sheath 100. The introducer sheath 100 may be formed by various polymeric or metallic materials. In further embodiments, the introducer sheath 100 may comprise an additional surface coating. The surface coating may include, but is not limited to, silicone, PET, or any other applicable polymer. A hub 120 is commonly included at the proximal opening (not shown). The hub 120, also referred to herein as a hemostasis valve hub, is configured for hemostasis, i.e, to prevent blood from leaking out of the introducer sheath during use. In various instances, it may be desired for the passage of multiple medical devices through the introducer sheath 100 at one time. As such, in some embodiments, as will be disclosed further with reference to FIGS. 3-8, the hemostasis valve hub 120 may include at least two openings and a manifold for allowing the insertion of at least two devices into the introducer sheath 100 at once, without requiring removal of the first device and subsequent insertion of the second device. The hub 120 provides a manifold structure for allowing the insertion of the two devices while also maintaining hemostasis and reducing the blood leakage from the introducer sheath 100 and/or the hub 120 during the insertion, use of, or removal of the various medical devices. In some embodiments, two catheters are inserted into the hub 120 at once, and various other medical devices may be inserted into at least one of the catheters for delivery into the blood vessel V. As shown in FIG. 1, the hub 120 may be used to receive a catheter 170. The catheter 170 extends through the hub 120 and the introducer sheath 100 and may couple to a proximal end of a blood pump which may be inserted into the introducer sheath 100, as will be described further herein.

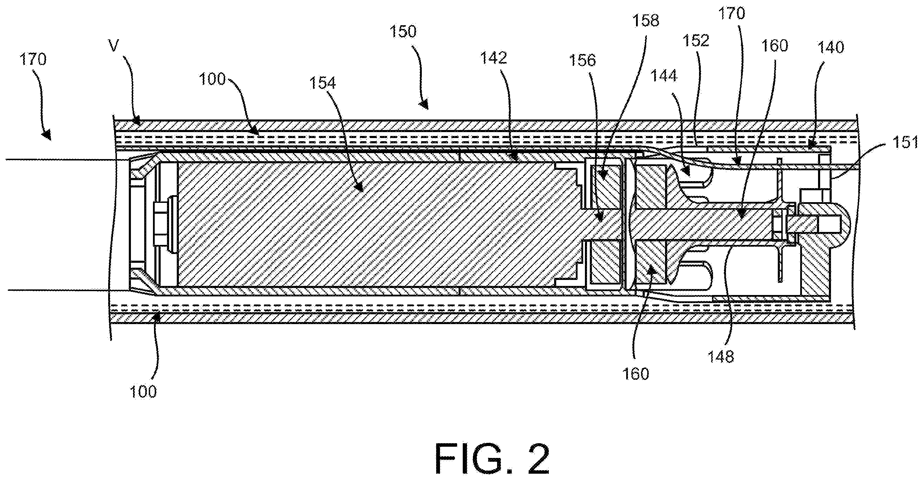

FIG. 2 illustrates a cross-sectional view of the introducer sheath 100 of FIG. 1 after insertion of a medical device, illustratively a blood pump 150, into the introducer sheath 100. The blood pump 150 generally includes an impeller assembly housing 140 and a motor housing 142. In some embodiments, the impeller assembly housing 140 and the motor housing 142 may be integrally or monolithically constructed. The impeller assembly housing 140 carries an impeller assembly 144 therein. The impeller assembly 144 includes an impeller shaft 146 and an impeller 148 that rotates relative to the impeller assembly housing 140 to drive blood through the blood pump 150. More specifically, the impeller 148 causes blood to flow from a blood inlet 151 formed on the impeller assembly housing 140, through the impeller assembly housing 140, and out of a blood outlet 152 formed on the impeller assembly housing 140. In some embodiments, the impeller shaft 146 and the impeller 148 may be integrated, and in other embodiments the impeller shaft 146 and the impeller 148 may be separate components. As shown in FIG. 2, the inlet 151 may be formed on an end portion of the impeller assembly housing 140 and the outlet 152 may be formed on a side portion of the impeller assembly housing 140. In other embodiments, the inlet 151 and/or the outlet 152 may be formed on other portions of the impeller assembly housing 140. In some embodiments, the impeller assembly housing 140 may couple to a distally extending cannula (not shown), and the cannula may receive and deliver blood to the inlet 151.

With continued reference to FIG. 2, the motor housing 142 carries a motor 154, and the motor 154 is configured to rotatably drive the impeller 148 relative to the impeller assembly housing 140. In the illustrated embodiment, the motor 154 rotates a drive shaft 156, which is coupled to a driving magnet 158. Rotation of the driving magnet 158 causes rotation of a driven magnet 160, which is connected to the impeller assembly housing 140. More specifically, in embodiments incorporating the impeller shaft 146, the impeller shaft 146 and the impeller 148 are configured to rotate with the driven magnet 160. In other embodiments, the motor 154 may couple to the impeller assembly housing 140 via other components. Additionally, as illustrated in FIG. 2, the catheter 170 extends from a proximal end of the blood pump 150. In some embodiments, the catheter 170 may be coupled to the motor housing 142 through a tapering connector and/or various other connecting means. The catheter 170 may have a flexible construction to facilitate the delivery of the blood pump 150. While the introducer sheath 100 is illustrated with the use of the blood pump 150, various other medical devices may be used in conjunction with the introducer sheath 100 and the hemostasis valve hub 120. For example, a variation of a blood pump may be used in conjunction with the introducer sheath 100. In other examples, a device other than a blood pump may be incorporated.

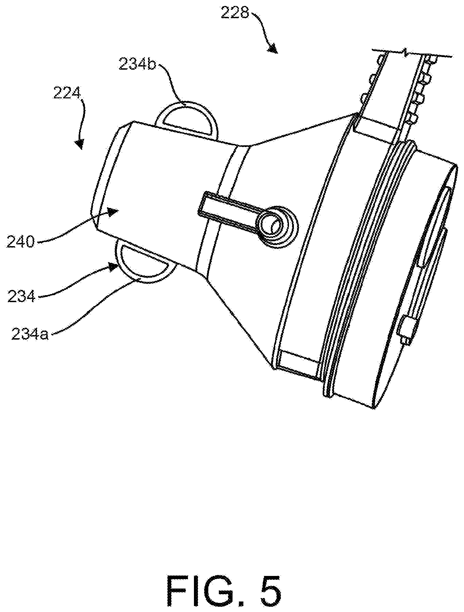

FIG. 3 illustrates an embodiment of a hemostasis valve hub 220. The hemostasis valve hub 220 includes a proximal end 222 and a distal end 224 positioned opposite the proximal end 222. The hemostasis valve hub 220 includes a hub cap 226 configured to be coupled to a hub base 228. As illustrated, the hub cap 226 has a first protruding portion 236 that extends from a face 238 of the hub cap 226. The first protruding portion 236 may be configured to couple to a tightening port (not shown) and used to secure a medical device, such as the catheter 170 (FIG. 2), passing through hub 220, for example to inhibit axial movement of the catheter 170. Additionally, the hub base 228 includes an access port 230 extending from the hub base 228. The access port 230 may be used for receiving additional fluids and/or medical devices. The hub base 228 comprises at least one flange 232 that may be used for grasping the hemostasis valve hub 220. In some embodiments, the hemostasis valve hub 220 comprises two or more flanges 232. The hemostasis valve hub 220 may also include one or more suture rings 234, as described in more detail with respect to FIG. 5.

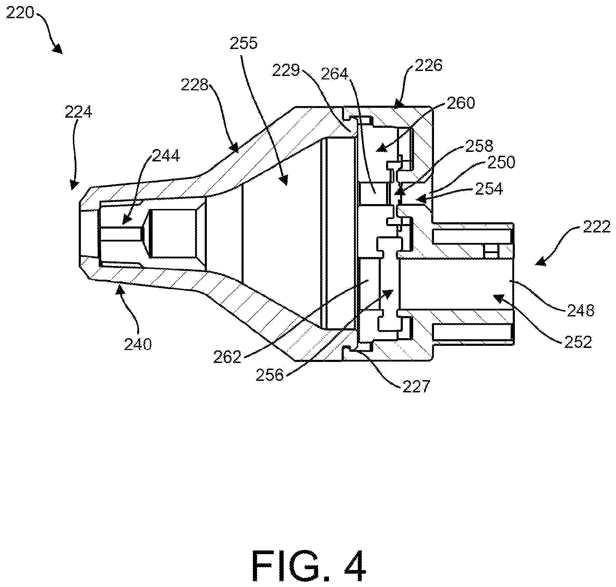

FIG. 4 illustrates a cross sectional view of the hemostasis valve hub 220 illustrated in FIG. 3. As illustrated, the hub cap 226 is engaged with the hub base 228. More specifically, the hub cap 226 comprises a plurality of engagement features 227, illustratively detents, for engaging with a plurality of engagement features 229 of the hub base 228, illustratively protrusions. The face 238 comprises a first cavity or opening 248 and a second cavity or opening 250. The first opening 248 extends into the first protruding portion 236 to define a first lumen 252 of the hemostasis valve hub 220. The second opening 250 defines a second lumen 254 of the hemostasis valve hub 220. Each of the first lumen 252 and the second lumen 254 are configured for receiving a medical device for insertion into the blood vessel V (FIG. 1). As described in more detail below, first opening 248 may be configured to receive relatively larger medical devices, such as the blood pump 150 with the catheter 170 (FIG. 2), while the second opening 250 is configured to receive relatively smaller medical devices, such as a catheter.

The first opening 248 comprises a primary seal 256 positioned extending radially across the first lumen 252, the second lumen 254 comprises a secondary seal 258 positioned extending radially the second lumen 254. Adjacent the primary seal 256 and the secondary seal 258 is a seal manifold 260. The seal manifold 260 comprises a first opening 262 aligned with the first opening 248 of the hub cap 226 and a second opening 264 aligned with the second opening 250 of the hub cap 226 to allow the first lumen 252 and the second lumen 254 to extend through the hub cap 226 and the hub base 228. Additionally, each of the first lumen 252 and the second lumen 254 extend into a main cavity 255 of the valve hub 220. In this way, when the one or more medical devices are inserted through the first lumen 252 and/or the second lumen 254, the medical device may extend through the main cavity 255 of the hub 220. Specifically, the first opening 262 and the first lumen 252 may be larger than the second opening 264 and the second lumen 254, configured such that the first opening 262 and the first lumen 252 are capable of receiving the relatively large medical devices, such as the blood pump 150 (FIG. 1). Additionally, the second opening 264 and the second lumen 254 may then be used for receiving other relatively smaller medical devices, such as guide catheters.

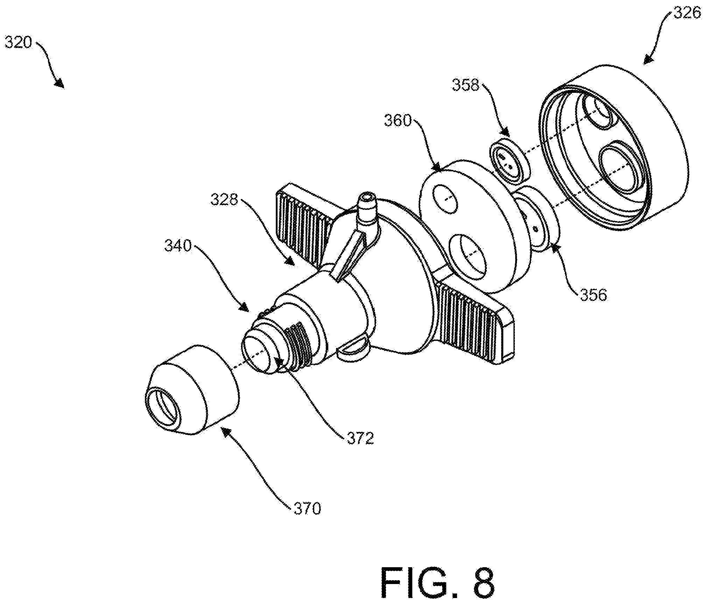

With continued reference to FIG. 4, toward the distal end 224 of the hub base 228, the hub base 228 comprises a narrowed portion 240 that includes or engages with a securing element for securing the hemostasis valve hub 220 to the introducer sheath 100. Additionally, the introducer sheath 100 (FIG. 1) is positioned around and engaged with the securing element. More specifically, the proximal end 106 (FIG. 1) of the introducer sheath 100, which has a flared portion, is secured by securing element to maintain the positioning of the introducer sheath 100. In the illustrative embodiment of FIG. 4, the securing element is a ferrule 244 configured to secure the hemostasis valve hub 220 at the flared portion of the proximal end 106 of the introducer sheath 100. However, in other embodiments, various other securing mechanisms may be used, as will be described with reference to FIG. 8.

FIG. 5 illustrates an enlarged view of the hub base 228, specifically illustrating the narrowed portion 240. As illustrated, the narrowed portion 240 includes at least one suture ring 234 that extends from the narrowed portion 240. The at least one suture ring 234 may be used for receiving a suture to secure the hemostasis valve hub 220 onto a patient. As illustrated in FIG. 5, the at least one suture ring 234 includes a first suture ring 234a and a second suture ring 234b, however any number of suture rings may be incorporated. Additionally, while illustrated as generally semi-circular or semi-oval rings, the suture rings 234 may have a variety of shapes incorporated.

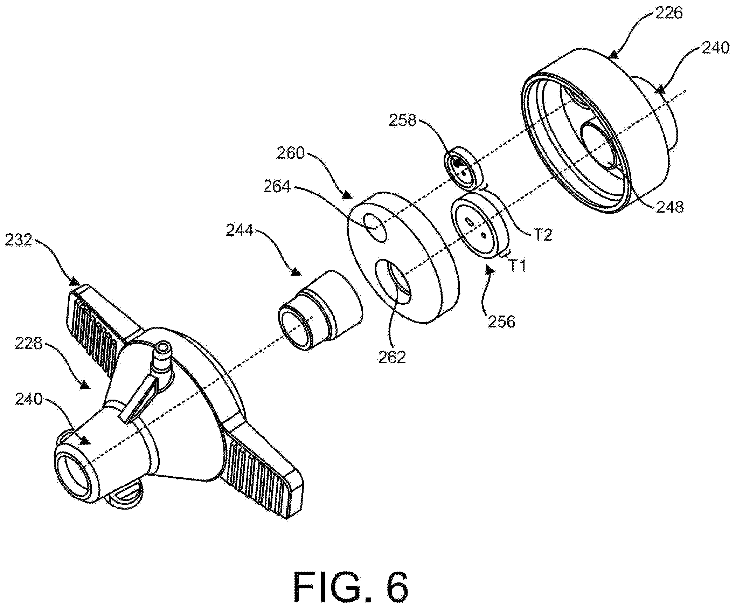

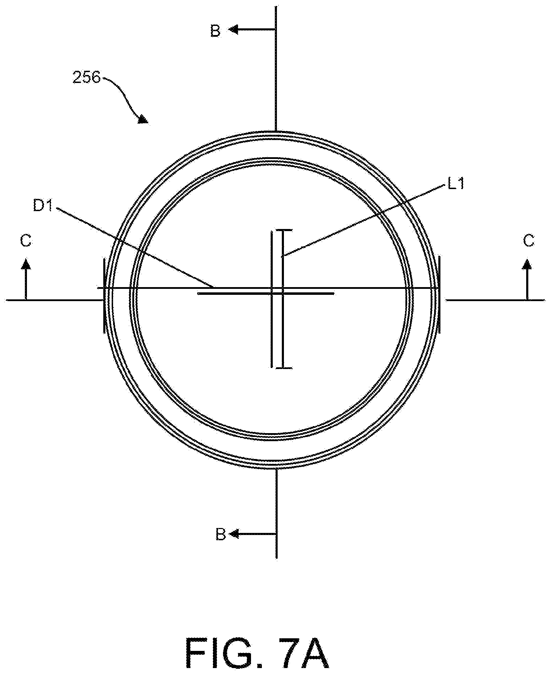

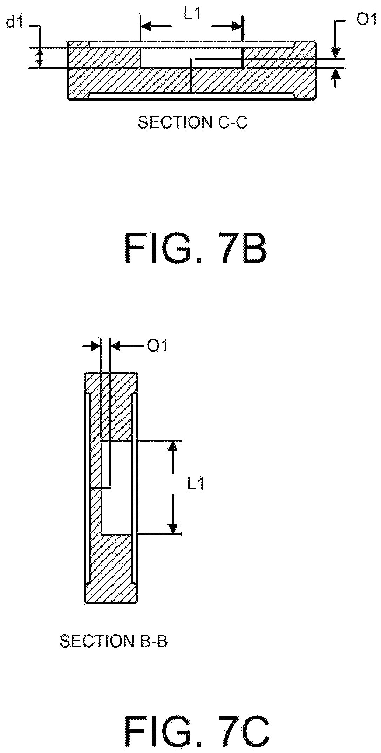

FIG. 6 illustrates an exploded view of the hemostasis valve hub 220 shown in FIGs. 3-6. As illustrated, during assembly, after the introducer sheath 100 (FIG. 1) has been inserted into the hub base 228, the ferrule 244 may be positioned within the narrowed portion 240 of the hub base 228. The seal manifold 260 may then be positioned adjacent the hub base 228, such that a first opening 262 of the seal manifold 260 aligns with the primary seal 256 and a second opening 264 of the seal manifold 260 aligns with the secondary seal 258. The first opening 262 of the seal manifold 260 has a diameter that is greater than a diameter of the second opening 264 of the seal manifold 260. As will be illustrated further with reference to FIGs. 7A-7B, the primary seal 256 comprises a first diameter D1 (FIG. 7A) that is greater than a second diameter D2 (FIG. 7B) of the secondary seal 258. Further, the first opening 248 of the hub cap 226 may have a diameter that is greater than a diameter of the second opening 250. As a result of the above-described configuration, the first opening 248 of the hub cap 226 and the first opening 262 of the seal manifold 260 are configured for complete radial coverage by the primary seal 256 such that there is a fluid seal between the seal manifold 260 and the hub base 228. Similarly, the second opening 264 of the seal manifold 260 and the second opening 250 of the hub cap 226 are configured for complete radial coverage by the secondary seal 258 such that there is a fluid tight seal between the seal manifold 260 and the hub base 228.

Additionally, the above-described configuration and alignment allows for the first and second lumens 252, 254 to extend continuously through the hemostasis valve hub 220. As such, a medical device, for example the blood pump 150 (FIG. 2) with the catheter 170 (FIG. 2) attached to the blood pump 150, may be inserted into the first lumen 252, extend through the main cavity 255 of the hub base 228, enter the proximal opening (not shown) of the introducer sheath 100, and pass beyond the distal end 224 of the hub base 228 within the lumen 112 of the introducer sheath 100. Similarly, an additional medical device, for example an additional catheter, may be inserted through the second lumen 254, extend through main cavity 255 of the hub base 228, enter the proximal opening of the introducer sheath 100, and pass beyond the distal end 224 of the hub base 228 within the introducer sheath 100. When inserted through the hemostasis valve hub 220, the medical devices extend through partial cross slits of the primary and secondary seals 256, 258, to contribute to the fluid tight and hemostatic seal of the introducer sheath 100 (FIG. 1) and the hemostasis valve hub 220.

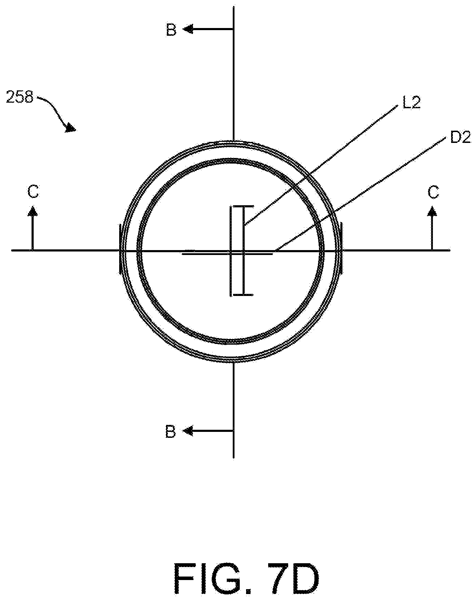

With reference to FIGs. 6-7F, the primary seal 256 and the secondary seal 258 are illustrated enlarged and described in further detail, respectfully. Specifically, FIG. 7A illustrates a top view of the primary seal 256 and FIG. 7D illustrates a top view of the secondary seal 258. As previously disclosed, the primary seal 256 may have a diameter D1 that is larger than a diameter D2 of the secondary seal 258. For example, the diameter D1 of the primary seal 256 may have a value of approximately 11 mm while the diameter D2 of the secondary seal 258 may have a value of approximately 8 mm. Additionally, the primary seal 256 may have a thickness T1 (FIG. 6) that is larger than a thickness T2 (FIG. 6) of the secondary seal 258. In various embodiments, the thickness T1 may have a value of approximately 2.0 mm while the thickness T2 may have a value of approximately 1.5 mm. However, the above-described dimensions of the primary seal 256 and the secondary seal 258 may vary. For example, the diameter D1 may range from 9 mm to 11 mm, and the diameter D2 may range from 5 mm to 8.5 mm. Additionally, the thickness T1 may range from 1.5 mm to 2.5 mm, and the thickness T2 may range from 1.25 mm to 2.00 mm.

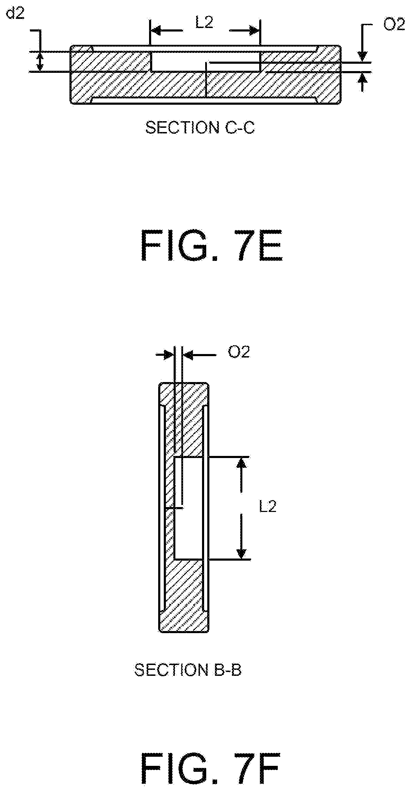

As illustrated in FIGs. 7A and 7D, respectfully, the primary and secondary seals 256, 258 may each be a cylindrical seal such that the primary and secondary seals 256, 258 have a circular cross-section. However, the configuration of the seals 256, 258 may vary. For example, the seals 256, 258 may be oval in cross section. Further, each of the primary seal 256 and the secondary seal 258 has a partial cross slit within the center of the seals 256, 258. The partial cross slit of the primary seal 256 may have a length L1 with a value of approximately 4.5 mm while the partial cross slits of the secondary seal 258 may have a length L2 with a value of approximately 2.5 mm. However, the lengths L1, L2 may be varied. For example, length L1 may range from 3.0 mm to 4.5 mm and the length L2 may range from 1.5 mm to 3.0 mm. Fig. 7B illustrates a cross sectional view of the primary seal 256 of FIG. 7A taken along line C-C, and FIG. 7C illustrates a cross sectional view of the primary seal 256 take along line B-B. As illustrated in FIGs. 7B and 7C, the partial cross slit forms when the primary seal 256 is slit cut at 90 degree orientation on opposite faces with a defined slit cut depth d and a defined overlap O**.** As illustrated, the partial cross slit of the primary seal 256 may have an overlap O1 with a value of approximately 0.4 mm and a slit depth d1 approximately 0.9 mm. Fig. 7E illustrates a cross sectional view of the secondary seal 258 of FIG. 7D taken along line C-C and Fig. 7F illustrates a cross sectional view of the secondary seal 258 taken along line B-B of Fig. 7D. The secondary seal 258 partial cross slit may be formed from slit cutting the secondary seal 258 at a 90 degree orientation on opposite faces with a slit cut depth d2 and a defined overlap O2. For example, the partial cross slits of the secondary seal 258 may have a value of the overlap O2 of approximately 0.25 mm and the slit depth d2 value may be approximately 0.80 mm. However, the values of the overlaps O1, O2 and the depths d1, d2 may be varied. For example, the overlap O1 may range from 0.3 mm to 0.5 mm, the depth d1 may range from 0.7 mm to 1.1 mm, the overlap O2 may range from 0.15 mm to 0.35 mm and the depth d2 may range from 0.6 mm to 1.0 mm. The partial cross slits are configured such that upon insertion of a catheter or other medical device through the seals 256, 258, the partial slits maintain a fluid tight seal around the circumference of the device. The primary seal 256 and the secondary seal 258 may each be composed of silicone, or various other suitable materials including polymer, thermoset, rubber or thermoset elastomer (TSE), or silicone rubber.

As a result of the above-described features of the hub cap 226, the seal manifold 260 and the primary and secondary seals 256, 258, the first lumen 252 has a larger diameter than the second lumen 254. As such, the first lumen 252 may be configured for receiving a larger medical device. For example, the first lumen 252 may be configured for receiving the blood pump 150, or another suitable medical device, while the second lumen 254 is configured for receiving a smaller medical device, for example a guide catheter, for navigation tools to be inserted into, or any other suitable device.

FIG. 8 illustrates an exploded view of an additional embodiment of a hemostasis valve hub 320. The hemostasis valve hub 320 may be similar to, or the same as, the hemostasis valve hub 220 as described with reference to FIGS. 3-7, with the exception of the securing mechanism. For example, the hemostasis valve hub 320 comprises a hub cap 326, a primary seal 356, a secondary seal 358, and a seal manifold 360 similarly to as described with reference to FIGs. 3-7. However, the hemostasis valve hub 320 comprises a modified hub base, illustratively hub base 328. The hub base 328 comprises a narrowed portion 340 with a distal end portion 372 that is configured for engaging with a securing mechanism. The distal end portion 372 may be threaded for engagement with a securing mechanism. Specifically, hemostasis valve hub 320 illustrates a threaded cap 370 that engages with the distal end portion 372 of the hemostasis valve hub 320. The threaded cap 370 may then engage with the proximal end 106 (FIG. 1) of the introducer sheath 100 to secure the introducer sheath 100 within the hemostasis valve hub 320. In particular, threaded cap 370 is used to prevent axial movement of the introducer sheath 100 with respect to hemostasis valve hub 320. In these instances, the proximal end 106 (FIG. 1) of the introducer sheath may have a flared configuration to better engage with the narrowed portion 340 and the securing mechanism.

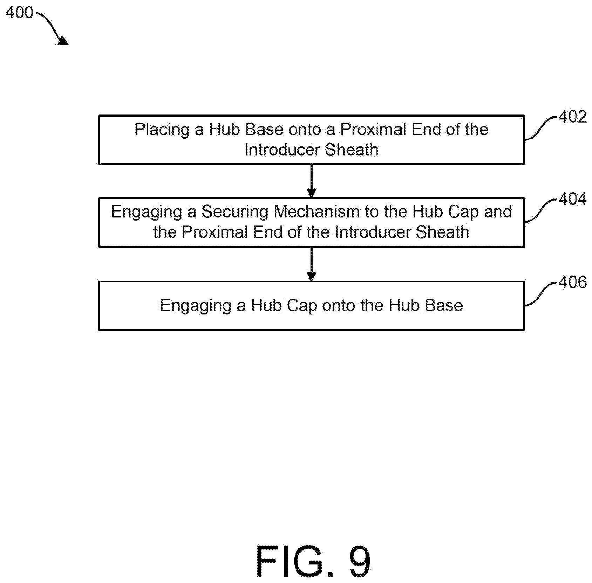

FIG. 9 illustrates a flow chart of a method 400 of assembling an introducer sheath and a hub, for example assembling the introducer sheath 100 of FIG. 1 with the hemostasis valve hub 220 as described with reference to FIGs. 3-7. However, the method 400 may also be used with the hemostasis valve hub 320 as described with reference to FIG. 8.

At block 402, the method 400 includes placing the hub base 228 onto the proximal end 106 of the introducer sheath 100 by inserting the introducer sheath 100 through the main cavity 255 of the hub base 228. More specifically, the introducer sheath 100 is placed into the hub base 228 until the proximal end 106 (FIG. 1) of the introducer sheath 100 is located within the narrowed portion 240 of the hub base 228. As previously described, the proximal opening (not shown) of the introducer sheath 100 may be on the flared portion of the proximal end 106 of the introducer sheath 100. In this way, the proximal end 106 of the introducer sheath 100 may extend though the distal end of the hub base 228 and into the blood vessel V (FIG. 1) during use. At block 404, the method 400 further includes engaging a securing mechanism to the hub base 228 and the proximal end 106 of the introducer sheath 100. In some embodiments, the securing mechanism is the ferrule 244. In these embodiments, after the introducer sheath 100 is positioned within the narrowed portion 240 of the hub 220, the ferrule 244 is inserted into the narrowed portion 240 of the hub base 228 to secure the introducer sheath 100 against an inner surface of the hub base 228. However, as previously described, the method 400 may be used with the hemostasis valve hub 320 and as such, the securing mechanism may be the threaded cap 370 as described with reference to FIG. 8.

At block 406, the method 400 further includes engaging the hub cap 226 onto the hub base 228. This step of engaging the hub cap 226 onto the hub base 228 may further include aligning the first opening 262 of the seal manifold 260 with the primary seal 256 and aligning the second opening 264 of the seal manifold 260 with the secondary seal 258. This may further include aligning the first opening 248 of the hub cap 226 with the primary seal 256 and aligning the second opening 250 of the hub cap 226 with the secondary seal 258. In this way, the alignment of the above described components allows for the first lumen and the second lumen to extend through the hemostasis valve hub 220 to receive various medical devices.

After assembly of the introducer sheath 100 and the hub 220, the introducer sheath 100 and the hub 220 may be used for delivery of at least one medical device into a blood vessel. More specifically, the introducer sheath 100 and the hub 220 can be used for inserting at least one catheter into the blood vessel V. Specifically, a physician or an operator may insert the introducer sheath 100 into the blood vessel V. The physician and the operator may then insert a medical device, for example a catheter, through the first opening 248 of the hub cap 226 and thus into the hub base 228 within the first lumen 252 of the hemostasis valve hub 220. In this way, the catheter extends through the first lumen 252 and within the primary seal 256. The catheter may then extend entirely through the hemostasis valve hub 220, out of the distal end 224 of the hemostasis valve hub 220, and into the introducer sheath 100. Due to the configuration of the hub 220, and more specifically the configuration of having both the first opening 248 with the primary seal 256 and the second opening 250 and the secondary seal 258, alongside the first medical device, a second medical device may be inserted into the hub 220 and the introducer sheath 100 and into the blood vessel 100. For example, the second medical device may be inserted through the second opening 250 of the hub cap 226 and extend though the second lumen 254 of the hemostasis valve hub 220. In this way, the second medical device extends through the secondary seal 258 to ensure a hemostatic sealing between the device and the hub 220. The second medical device can then be extended through the hemostasis valve hub 220 and out of the distal end 224 of the hemostasis valve hub 220 for insertion into the introducer sheath 100.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the above-described features.

Although the present invention is defined in the attached claims, it should be understood that the present invention can also (alternatively) be defined in accordance with the following embodiments:
1. A hemostasis valve hub for an introducer sheath, comprising:
   a hub base;
   a hub cap configured for engagement with the hub base, the hub cap having a first wall with a first opening and a second opening extending through the first wall,
   a manifold positioned within the hub cap and adjacent the hub base;
   a primary seal positioned adjacent the first opening of the hub cap;
   a secondary seal positioned adjacent the second opening of the hub cap; and
   wherein the primary seal and the secondary seal are configured to provide a fluid seal between the first opening and the second opening, respectively, and the hub base.
2. The hemostasis valve hub of Embodiment 1, wherein the first opening has a first diameter and the second opening has a second diameter, the first diameter being greater than the second diameter.
3. The hemostasis valve hub of any one of Embodiments 1-2, wherein the primary seal has a first thickness, the secondary seal has a second thickness, and the first thickness is greater than the second thickness.
4. The hemostasis valve hub of any one of Embodiments 1-3, wherein the hub base comprises a distal end and a proximal end and wherein the hub base has a ferrule positioned within the distal end of the hub base.
5. The hemostasis valve hub of Embodiment 4, wherein the ferrule is configured for securing the introducer sheath with the hub base.
6. The hemostasis valve hub of any one of Embodiments 1-3, wherein the hub base comprises a distal end and a proximal end and wherein a threaded cap is configured for attachment to the distal end of the hub base for securing the introducer sheath with hub base.
7. The hemostasis valve hub of any one of Embodiments 1-6, wherein the primary seal and the secondary seal are each composed of cylindrical seals having a partial cross slit within a center of each the first seal and the second seal.
8. The hemostasis valve hub of any one of Embodiments 1-7, wherein the hub base comprises at least one ring configured for receiving a suture to secure the hemostasis valve hub positioning.
9. A delivery system for a plurality of medical devices into a blood vessel, comprising:
   an introducer sheath for insertion into the blood vessel, the introducer sheath having a proximal end and a distal end; and
   a hemostasis valve hub for attachment to the proximal end of the introducer sheath, the hemostasis valve hub including:
      a hub base;
      a hub cap configured for engagement with the hub base, the hub cap having a first face with a first opening and a second opening extending through the first face,
      a manifold positioned within the hub cap and adjacent the hub base;
      a primary seal positioned adjacent the first opening of the hub cap;
      a secondary seal positioned adjacent the second opening of the hub cap; and
      wherein the primary seal and the secondary seal are configured to provide a fluid seal between the first opening and the second opening, respectively, and the hub base.
10. The delivery system of Embodiment 9, wherein the first opening has a first diameter and the second opening has a second diameter, the first diameter being greater than the second diameter.
11. The delivery system of any one of Embodiments 9-10, wherein the hub base has at least one ring for receiving a suture.
12. The delivery system of any one of Embodiments 9-11, wherein the hub base comprises a distal end and a proximal end and wherein the hub base has a ferrule positioned within the distal end of the hub base.
13. A method of assembling an introducer sheath and a hub, comprising:
   placing a hub base onto the proximal end of the introducer sheath;
   attaching a securing mechanism to the hub base and the proximal end of the introducer sheath; and
   engaging a hub cap onto the hub base, the hub cap having a seal manifold positioned within the hub cap and a first face having a first opening and a second opening, a first seal positioned between the seal manifold and the first opening and a second seal positioned between the seal manifold and the second opening.
14. The method of Embodiment 13, wherein engaging the hub cap with the hub base includes aligning a first opening of the seal manifold with the primary seal and aligning a second opening of the seal with the secondary seal.
15. The method of Embodiment 14, wherein the first opening of the seal manifold and the first opening of the hub cap define a first lumen, and the second opening of the seal manifold and the second opening of the hub cap define a second lumen.

## Claims

1. A hemostasis valve hub (220, 320) for an introducer sheath (100), comprising:
a hub base (228, 328);
a hub cap (226, 326) configured for engagement with the hub base, the hub cap having a first wall with a first opening (248, 348) and a second opening (250, 350) extending through the first wall,
a manifold (260, 360) positioned within the hub cap and adjacent the hub base;
a primary seal (256, 356) positioned adjacent the first opening of the hub cap;
a secondary seal (258, 358) positioned adjacent the second opening of the hub cap; and
wherein the primary seal and the secondary seal are configured to provide a fluid seal between the first opening and the second opening, respectively, and the hub base,
wherein the first opening (248, 348) is configured to receive a blood pump (150).

2. The hemostasis valve hub (220, 320) of Claim 1, wherein the first opening has a first diameter (D1) and the second opening has a second diameter (D2), the first diameter (D1) being greater than the second diameter (D2).

3. The hemostasis valve hub (220, 320) of any one of Claims 1-2, wherein the primary seal has a first thickness (T1), the secondary seal has a second thickness (T2), and the first thickness (T1) is greater than the second thickness (T2).

4. The hemostasis valve hub (220) of any one of Claims 1-3, wherein the hub base comprises a distal end and a proximal end and wherein the hub base has a ferrule (244) positioned within the distal end of the hub base, and wherein the ferrule is configured for securing the introducer sheath with the hub base.

5. The hemostasis valve hub (320) of any one of Claims 1-3, wherein the hub base (328) comprises a distal end and a proximal end and wherein a threaded cap (370) is configured for attachment to the distal end of the hub base for securing the introducer sheath with hub base.

6. The hemostasis valve hub (320) of any one of Claims 1-5, wherein the hub base (228) includes an access port (230) extending from the hub base (228), and wherein the hub base (228) comprises at least one flange (232) configured for grasping the hemostasis valve hub (320).

7. The hemostasis valve hub (220, 320) of any one of Claims 1-6, wherein the primary seal and the secondary seal are each composed of cylindrical seals having a partial cross slit within a center of each the primary seal and the secondary seal.

8. The hemostasis valve hub (220, 320) of any one of Claims 1-7, wherein the hub base comprises at least one ring (234) configured for receiving a suture to secure the hemostasis valve hub positioning.

9. The hemostasis valve hub (220, 320) of any one of Claims 1-8, wherein the first opening (248, 348) is configured to receive the blood pump (150) with a catheter (170) extending from a proximal end of the blood pump (150), and
wherein the second opening (250) is configured for receiving a medical device smaller than the blood pump (150) with the catheter (170), such as an additional catheter or a guide catheter, or for insertion of navigation tools.

10. The hemostasis valve hub (220, 320) of any one of Claims 1-9, wherein the hub cap (226) has a first protruding portion (236) that extends from a face (238) of the hub cap (226), wherein the first protruding portion is configured to couple to a tightening port.

11. The hemostasis valve hub (220, 320) any one of Claims 1-10, wherein the manifold (260) is a seal manifold that comprises a first opening (262) aligned with the first opening (248) of the hub cap (226) to define a first lumen (252) of the hemostasis valve hub (220, 320) and a second opening (264) aligned with the second opening (250) of the hub cap (226) to define a second lumen (254) of the hemostasis valve hub (220, 320),
wherein the first lumen (252) is larger than the second lumen (254),
wherein the primary seal (256, 356) is positioned extending radially across the first lumen (252) and wherein the secondary seal (258, 358) is positioned extending radially across second lumen (254).

12. A delivery system for a plurality of medical devices into a blood vessel, comprising:
an introducer sheath (100) for insertion into the blood vessel, the introducer sheath having a proximal end (106) and a distal end (108); and
a hemostasis valve hub (220, 320) according to any one of the preceding claims for attachment to the proximal end of the introducer sheath.

13. A method (400) of assembling an introducer sheath (100) and a hub (220, 320), comprising:
placing (402) a hub base (228, 328) onto a proximal end (106) of the introducer sheath;
attaching (404) a securing mechanism to the hub base and the proximal end of the introducer sheath; and
engaging (406) a hub cap (226, 326) onto the hub base, the hub cap having a seal manifold (260, 360) positioned within the hub cap and a first face having a first opening (248, 348) and a second opening (250, 350), wherein the primary seal is positioned between the seal manifold and the first opening and wherein the secondary seal is positioned between the seal manifold and the second opening,
wherein the first opening (248, 348) is configured to receive a blood pump (150).

14. The method (400) of Claim 13, wherein engaging the hub cap with the hub base includes aligning a first opening (262) of the seal manifold with the primary seal and aligning a second opening (264) of the seal with the secondary seal.

15. The method (400) of Claim 14, wherein the first opening of the seal manifold and the first opening of the hub cap define a first lumen (252), and the second opening of the seal manifold and the second opening of the hub cap define a second lumen (254).
